# EUROPÄISCHE PATENTSCHRIFT

(11) **EP 2 856 003 B1**
(45) Veröffentlichungstag und Bekanntmachung des Hinweises auf die Patenterteilung: **30.11.2016**
(21) Anmeldenummer: 13782648.3
(22) Anmeldetag: 22.08.2013
(51) Int. Cl.: F17D 1/16, F17D 1/17, B01F 17/00, C07C 29/151, C07C 41/09, C07C 41/42, C07C 41/34, C07C 29/20

(54) **VERFAHREN ZUR VERBESSERUNG DER TRANSPORTFÄHIGKEIT VON SCHWEREM ROHÖL**
METHOD FOR IMPROVING THE TRANSPORTABILITY OF HEAVY CRUDE OIL
PROCÉDÉ D'AMÉLIORATION DE L'APTITUDE À L'ÉCOULEMENT DE PÉTROLE BRUT LOURD

(30) Priorität: 04.09.2012 WO PCT/DE2012/100262
(43) Veröffentlichungstag der Anmeldung: 08.04.2015
(73) Patentinhaber: Wagner, Ulrich, 06406 Bernburg (DE); Balthasar, Wolff, 40833 Ratingen (DE); Müller, Dierk, 61184 Karben (DE)
(72) Erfinder: Wagner, Ulrich, 06406 Bernburg (DE); Balthasar, Wolff, 40833 Ratingen (DE); Müller, Dierk, 61184 Karben (DE)
(74) Vertreter: Söylemezoglu, Mustafa Nazim
(86) Internationale Anmeldenummer: PCT/DE2013/100302
(87) Internationale Veröffentlichungsnummer: WO 2014/036994

(56) Entgegenhaltungen:
- DE-A1- 3 504 231
- US-A- 3 870 063
- US-A- 4 076 761
- US-A1- 2005 197 412
- US-A1- 2009 014 336

## Beschreibung

Die Erfindung bezieht sich auf ein Verfahren zur Verbesserung der Transportfähigkeit von schwerem Rohöl.

Es ist allgemein bekannt, in einer Erdöllagerstätte gefördertes Erdöl als Rohöl zur weiteren Verarbeitung in einer Raffinerie über Rohrleitungen zu transportieren, wobei sich diese über Entfernungen von mehreren tausend Kilometern erstrecken können.

Lassen sich in einer Erdöllagerstätte nur noch schwere oder superschwere Rohöle gewinnen, also solche z.B. mit einer Viskosität von in weniger als 40 000 mPa s bzw. 24° API (API = amerikanische Dichteeinheit für Rohöl), so ist ein längerer Transport derartiger Rohöle durch Rohrleitungen ohne zusätzliche Maßnahmen nicht mehr möglich bzw. unwirtschaftlich.

Es wurde daher bereits nach verschiedenen Möglichkeiten gesucht, die Transportfähigkeit von schwerem Rohöl, insbesondere durch Verringerung der Viskosität, zu verbessern.

Aus der DE 36 09 641 A1 ist bekannt, zum Transport von zähfließendem Rohöl dieses in eine Öl-in-Wasser-Emulsion mit mindestens 10 bis 15% Wasser unter Zusatz eines speziellen Emulgators auf Basis Oxethylat umzuwandeln.

Gemäß WO 2011/006024 A2 wird zur Verringerung der Viskosität vorgeschlagen, ein Polymer bestehend aus einem nicht-ionischen Monomeren und mindestens 25 Molprozent kationischer Monomere einzusetzen.

Der Zusatz von Emulgatoren oder Polymeren als Verdünnungsmittel ist mit zusätzlichen Kosten verbunden und erfordert, dass diese vor der Raffination des Rohöls wieder entfernt werden müssen.

In der DE 2 039 329 A wird zur Transportverbesserung vorgeschlagen, Rohöl auf Temperaturen von 340 bis 650 °C zu erhitzen. Dies ist jedoch mit einem erheblichen Aufwand verbunden und bei Transportstrecken von mehreren tausend Kilometern wirtschaftlich nicht realisierbar.

Die US 3,870,063 schlägt vor, an der Förderstelle dem Rohöl einen Zusatz wie Benzin zur Verringerung der Viskosität zuzufügen. Dieser Zusatz soll dann am fernen Ende der Pipeline von dem Rohöl getrennt und über eine separate Pipeline oder mittels Transportfahrzeugen wieder an die Förderstelle zum Schließen des Kreislaufes zurückbefördert werden.

Die US 2005/0197412 A1 beschreibt ein Verfahren zur Herstellung eines Methanol-Gemisches mit erhöhtem Ethanolanteil aus Erdgas.

Die US 4,076,761 offenbart ein Verfahren zur Umwandlung von fossilen Brennstoffen in Motorenbenzin von hoher Güte.

Die US 2009/0014336 A1 zeigt ein Verfahren zur Herstellung von Methanol aus Kohlendioxid von Industrie- oder Kraftwerksabgasen oder aus der Atmosphäre.

In der US 7,861,737 82 wird vorgeschlagen, zur Transportverbesserung von Schweröl diesem erst ein Lösungsmittel, wie z.B. Naphtha, zuzusetzen, um das Schwer- bzw. Rohöl zu verdünnen. Danach wird Dimethylether (DME) in flüssigem oder gasförmigem Zustand unter hohem Druck, mindesten 4 bar, eingetragen. Der Zusatz von DME soll zu einer deutlichen Verringerung der Viskosität des Schwer- bzw. Rohöls führen, wodurch die Transportfähigkeit verbessert wird.

Der Nachteil dieser Lösung ist, dass zwei Komponenten, Naphtha und DME, bereitgestellt, zur Erdöllagerstätte transportiert und dem Schwer- bzw. Rohöl zugesetzt werden müssen. Der Zusatz von DME als Komponente mit einem hohen Partialdruck ist mit zusätzlichem Aufwand beim Mischen und Verpumpen verbunden. Naphtha enthält überwiegend Cycloparaffine. Die Zusätze Naphtha und DME müssen während der nachfolgenden Raffination des Rohöls destillativ mit abgetrennt werden. DME ist unter Normalbedingungen ein hochentzündliches Gas. Der Umgang mit diesem hochexplosiven Stoff erfordert erhebliche sicherheitstechnische Aufwendungen.

Aufgabe der Erfindung ist es, ein Verfahren zur Verbesserung der Transportfähigkeit von schwerem Rohöl mittels eines Zusatzes zu schaffen, der aus einem bei der Erdölgewinnung anfallenden Nebenprodukt herstellbar ist, keine besonderen sicherheitstechnischen Vorkehrungen erfordert, mit geringem Aufwand dem schweren Rohöl zugesetzt werden kann, während der nachfolgenden Raffination nicht abgetrennt werden muss und zu einer erhöhten Ausbeute an herkömmlichem Benzin bei der Raffination führt. Erfindungsgemäß wird die Aufgabe durch die im Anspruch 1 angegebenen Merkmale gelöst. Vorteilhafte Ausgestaltungen und Weiterbildungen der Verfahrensweise sind Gegenstand der Ansprüche 2 bis 18.

Erfindungsgemäß wird dem schweren Rohöl vor dessen Entgasung und Entwässerung als viskositätserniedrigendes Mittel ein wasserhaltiges Kohlenwasserstoffgemisch mit einer Kettenlänge von vorwiegend C4 bis C12, das keine sauerstoffhaltigen Kohlenwasserstoffverbindungen enthält, und im Bereich einer Erdöllagerstätte aus als Nebenprodukt anfallendem Erdgas und/oder Erdölbegleitgas gewonnen wird, zugesetzt.

Vorwiegend bedeutet hier, dass ca. 80 bis 85 % der Kohlenwasserstoffe eine Kettenlänge von C4 bis C12 aufweisen. Bei den restlichen 15 bis 20 % handelt es sich um Verbindungen mit den Kettellängen C3 bzw. >C12.

Die Einsatzmenge des Kohlenwasserstoffgemisches liegt vorzugsweise im Bereich von 20 bis 40 %, bezogen auf die Menge an Rohöl.

Das spezielle Kohlenwasserstoffgemisch wird vor Ort wie folgt hergestellt:
a) Umwandlung des Erdgases und/oder Erdölbegleitgases in ein Methanol/Wasser-Gemisch,
b) destillative Aufarbeitung des Methanol-Wasser-Gemisches zu einem Destillat mit einem hohen Wasser- und Alkoholgehalt von über 90 %,
c) katalytische Umwandlung des Destillates in ein Dimethylether/Methanol/Wasser-Gemisch,
d) Umwandlung des Dimethylether/Methanol/Wasser-Gemisches durch Dehydratation in das wasserhaltige Kohlenwasserstoffgemisch mit einer Kettenlänge C4 bis C12.

Das gemäß den Verfahrensschritten a) bis d) erhaltene Kohlenwasserstoffgemisch wird entweder unbehandelt oder nach Entgasung und/oder Entwässerung dem schweren Rohöl zugesetzt, wodurch aus dem schweren Rohöl ein in seiner Qualität leichtes Rohöl erhalten wird, das über Leitungen zu einer Raffinerie transportiert wird. Während der nachfolgenden Raffination des leichten Rohöls zu herkömmlichem Benzin wird die produzierte Menge an Benzin um die im wasserhaltigen Kohlenwasserstoffgemisch enthaltene Menge an Kohlenwasserstoffen erhöht.

Das Ausgangsprodukt, Erdgas und/oder Erdölbegleitgas, fällt bei der Gewinnung von schwerem Rohöl in Erdöllagerstätten, z.B. bei einer sogenannten Cluster-Förderung, an. Das Begleitgas wird mittels einer Fluidabtrenneinrichtung abgetrennt. Bisher war es übliche Praxis, das als Nebenprodukt anfallende Erd- bzw. Begleitgas zurück zu verpressen oder abzufackeln.

Von großem ökonomischem Vorteil ist einerseits die Verwertung des als Nebenprodukt anfallenden Erd- bzw. Begleitgases unmittelbar am Entstehungsort und andererseits die Tatsache, dass das zu fließfähigerem und transportfähigem Rohöl modifizierte schwere Rohöl keiner gesonderten Behandlung unterzogen werden muss. Es kann nunmehr in einer Raffinerie wie normales leichtes Rohöl weiterverarbeitet werden. Unter "leichtem Rohöl" sind dabei solche Rohöle zu verstehen, die einen API von in etwa 30° oder größer besitzen. Während der Raffination des leichten Rohöls stellt sich ein weiterer Vorteil ein. Das zugesetzte Kohlenwasserstoffgemisch enthält bereits Kohlenwasserstoffe im Bereich C4 bis C12, wie herkömmliches Benzin. Demzufolge erhöht sich bei der Raffination des leichten Rohöls die gewonnene Benzinmenge um ca. den Anteil an erfindungsgemäß zugesetztem Kohlenwasserstoffgemisch.

Dadurch lassen sich im Vergleich zu schwerem Rohöl deutlich höhere Verkaufserlöse erzielen. Der Aufwand für die Errichtung einer Anlage unmittelbar vor Ort zur chemischen Umsetzung von Erdgas bzw. Erdölbegleitgas in Kohlenwasserstoffgemisch amortisiert sich somit bereits nach relativ kurzer Betriebsdauer.

Die Umwandlung des Erd- und/oder Erdölbegleitgases in ein Methanol-Wasser-Gemisch kann nach zwei unterschiedlichen Verfahrensweisen erfolgen:
Gemäß einer ersten Variante sind folgende Verfahrensschritte vorgesehen:
   - Entschwefelung; Sättigung mit Prozesskondensat und Dampf;
   - Vorspaltung in ein Gasgemisch aus Methan, Kohlendioxid und Kohlenmonoxid;
   - anschließend wird das vorgespaltene Gasgemisch unter erhöhter Temperatur und bei einem Druck von mindestens 50 bar in einem Autothermreaktor katalytisch in Synthesegas unter Zusatz von vorgeheiztem Sauerstoff umgewandelt, das abgekühlt und mittels eines Kompressors komprimiert wird, und
   - nachfolgend aus diesem durch katalytische Umwandlung im Rahmen einer zweistufigen Wasser-Methanol-Synthese in einem wassergekühlten und in einem gasgekühlten Reaktor Methanol gewonnen und durch nachfolgende mehrstufige Kondensation Rohmethanol (Methanol-Wasser-Gemisch) erhalten wird.
Gemäß einer zweiten Variante sind folgende Verfahrensschritte vorgesehen:
   - Entschwefelung; Sättigung mit Prozesskondensat und Dampf;
   - nachfolgende Auskreisung eines Teilstromes an mit Wasser gesättigtem Prozessgas, das in ein Gasgemisch aus Methan, Wasserstoff, Kohlendioxid und Kohlenmonoxid vorgespalten wird;
   - dieses Gasgemisch wird in einem Steamreformer in ein erstes Synthesegas, ein Gemisch aus Wasserstoff, Kohlendioxid und Kohlenmonoxid, umgewandelt, das wieder in den mit Wasser gesättigten Prozessgasstrom zurückgeführt und mit diesem vermischt wird;
   - anschließend wird der Prozessgasstrom unter erhöhter Temperatur und bei einem Druck von mindestens 50 bar in einem Autothermreaktor unter Zusatz von vorgeheiztem Sauerstoff katalytisch in ein zweites Synthesegas umgewandelt, das abgekühlt und mittels eines Kompressors komprimiert wird, und
   - nachfolgend aus diesem durch katalytische Umwandlung im Rahmen einer zweistufigen Wasser-Methanol-Synthese in einem wassergekühlten und in einem gasgekühlten Reaktor Methanol gewonnen und durch nachfolgende mehrstufige Kondensation Rohmethanol (Methanol-Wasser-Gemisch) erhalten wird.

Das jeweils erhaltene Methanol-Wasser-Gemisch (Rohmethanol) wird nachfolgend einer zweitstufigen Destillation unterzogen, wobei in der ersten Stufe niedrig siedende und in der zweiten Stufe höher siedende Verbindungen abgetrennt werden und ein Destillat mit einem hohen Wasser- und Alkoholgehalt entsteht. Dieses wird nachfolgend in einem Festbettreaktor katalytisch in ein Dimethylether/Methanol/Wasser-Gemisch umgewandelt, das anschließend in weiteren adiabatisch arbeitenden Reaktoren im Temperaturbereich von 300 bis 450 °C in das wasserhaltige Kohlenwasserstoffgemisch als Endprodukt umgewandelt wird.

Dieses Kohlenwasserstoffgemisch hat beispielsweise folgende Zusammensetzung:
- 57 % Wasser
- 5 % Propan
- 38 % Kohlenwasserstoffe (hauptsächlich im Bereich C4 bis C12).

Die Kohlenwasserstoffe bestehen aus Paraffinen, Olefinen und Aromaten.

Das im Festbettreaktor anfallende Dimethylether/Methanol/Wasser-Gemisch wird vorzugsweise mit Recyclegas zur Temperatureinstellung versetzt.

Gemäß einer bevorzugten Ausführung wird eine erste Teilmenge an Synthesegas ausgekreist, im Kreislauf gefahren und dabei auf den erforderlichen Betriebsdruck komprimiert.

Es kann auch noch eine zweite Teilmenge an Synthesegas ausgekreist werden, aus der in einer Pressure-Swing-Anlage Wasserstoff abgetrennt wird, der auf der Saugseite des Kompressors wieder in den Synthesegasstrom eingeleitet wird.

Das unmittelbar am Förderort hergestellte Kohlenwasserstoffgemisch wird nunmehr entweder unbehandelt oder nach Entgasung und/oder Entwässerung dem schweren Rohöl zugesetzt, wobei dieses verdünnt und dadurch die Transportfähigkeit deutlich verbessert wird.

Über die Zusatzmenge an Kohlenwasserstoffgemisch lässt sich die Viskosität entsprechend der gewünschten Transportqualität gezielt einstellen. Zur Herabsetzung der Viskosität des schweren Rohöls werden in Abhängigkeit vom API-Grad bis zu 40 %, bezogen auf die Menge an schwerem Rohöl, zugesetzt. Dadurch wird eine für einen Transport ausreichend hohe Verdünnung erzielt.

Es können auch größere Mengen zugesetzt werden, die sich jedoch nur noch unwesentlich auf eine weitere Verringerung der Viskosität auswirken. Bereits kleine Zusatzmengen, im einstelligen Prozentbereich, können ausreichen, um die Qualität des schweren Rohöls zu verbessern. Vorzugsweise führen mindestens 10 %, bezogen auf die Menge an ungereinigtem Rohöl, zu sehr guten Ergebnissen.

Dem schweren Rohöl wird ein Kohlenwasserstoffgemisch mit einer Kettenlänge von vorwiegend C4 bis C12 zugesetzt, das keine sauerstoffhaltigen Kohlenwasserstoffverbindungen enthält.

Die speziellen Bedingungen zur Gewinnung dieses Kohlenwasserstoffgemisches sind im nachfolgenden Ausführungsbeispiel angegeben.

Das als Zwischenprodukt gebildete Methanol sollte vorzugweise noch einen Restwassergehalt von mindestens 4 % und einen Alkoholgehalt von 0,1 % besitzen. Es wird durch Dehydratation katalytisch zu einem wasser- und gashaltigen Kohlenwasserstoffgemisch umgesetzt.

Dieses Kohlenwasserstoffgemisch kann unmittelbar im Bereich der Erdöllagerstätte zur Verbesserung der Förderfähigkeit entweder dem bereits geförderten und/oder dem noch unterirdisch gelagerten schweren Rohöl über das Bohrloch zugeführt werden. Vorzugsweise erfolgt die Einbringung in ein Bohrloch über ein in dieses eingesetztes Spülrohr.

Bei einzelnen Schächten zur Förderung eines Erdöl-Clusters besteht auch die Möglichkeit, dass eine erste Teilmenge an Kohlenwasserstoffgemisch oberhalb des Bohrloches zugeführt wird, um die Transportfähigkeit des schweren Rohöls zu verbessern. Die Erdölschächte eines Clusters werden zusammengeführt, wobei nach dem Zusammenführen die Fluidströme vermischt und in einer Massenabtrenneinrichtung Wasser und Ölbegleitgas abgetrennt werden. Während des Vermischens kann wiederum eine entsprechende Menge an Kohlenwasserstoffgemisch zugesetzt werden. Dieses wird in Abhängigkeit von der Viskosität des Schweröls so dosiert, dass dessen Transportfähigkeit in ausreichendem Maße verbessert wird bis zu einer Qualität, wie leichtes Rohöl.

Erforderlichenfalls kann das gebildete Kohlenwasserstoffgemisch vor dem Inkontaktbringen mit dem schweren Rohöl noch gereinigt, also entwässert und entgast werden. Durch separate Wasserabtrennung und Entgasung kann aus dem wässrigen Kohlenwasserstoffgemisch ein wasserfreies Kohlenwasserstoffgemisch erzeugt werden. Grundsätzlich kann das aufbereitete Kohlenwasserstoffgemisch an jeder gewünschten Stelle zur Verbesserung der Förder- oder Transportfähigkeit zugesetzt werden. Gereinigtes Kohlenwasserstoffgemisch kann vorzugsweise auf der Saugseite der zum Transport des Rohöls eingesetzten Pumpe zugeführt werden. Gegebenenfalls können Kohlenwasserstoffgemisch und schweres Rohöl auch in einer separaten Mischeinrichtung zu leichtem Rohöl vermischt werden.

Einsatzmengen an Kohlenwasserstoffgemisch von ca. 20 % sind bereits ausreichend, um z.B. Schweröl (API 23°) in leichtes Rohöl (API 31°) umzuwandeln.

Vorzugsweise wird die Viskosität des geförderten schweren Rohöls gemessen und in Abhängigkeit vom aktuellen Messergebnis die Menge an Kohlenwasserstoffgemisch dosiert zugesetzt, um leichtes Rohöl zu erhalten.

Unaufbereitetes Kohlenwasserstoffgemisch muss innerhalb der Förder- und Transportstrecke des schweren Rohöls diesem vor Erreichen der Massenabtrenneinrichtung zugesetzt werden. Dagegen kann aufbereitetes Kohlenwasserstoffgemisch, das entwässert und entgast ist, an allen Stellen der Förder- und Transportstrecke dem schweren Rohöl zugesetzt werden.

Die Erfindung wird nachstehend an zwei Beispielen näher erläutert.

In der zugehörigen Zeichnung zeigen,
Fig. 1 eine erste Ausführungsvariante als Fließschema und
Fig. 2 eine zweite Ausführungsvariante als Fließschema.

In einer Erdöllagerstätte werden 1088 t/h an schwerem Rohöl (API 23°) gefördert, das folgende Zusammensetzung aufweist:

| | |
|---|---|
| Kohlenwasserstoffe | 818 t |
| Wasser | 240 t und |
| gasförmige Bestandteile | 30 t. |

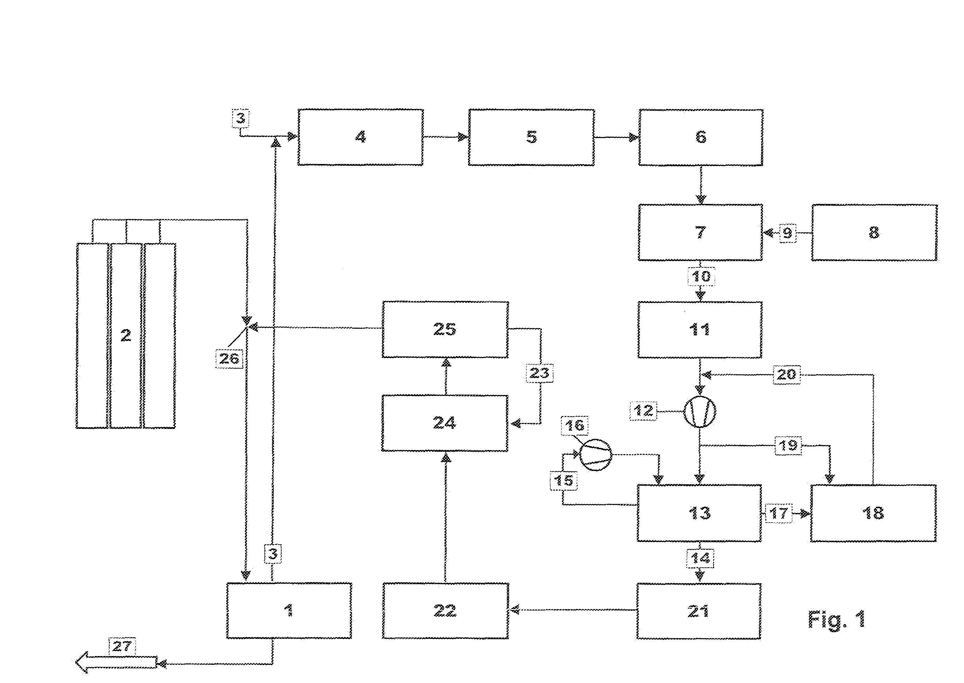

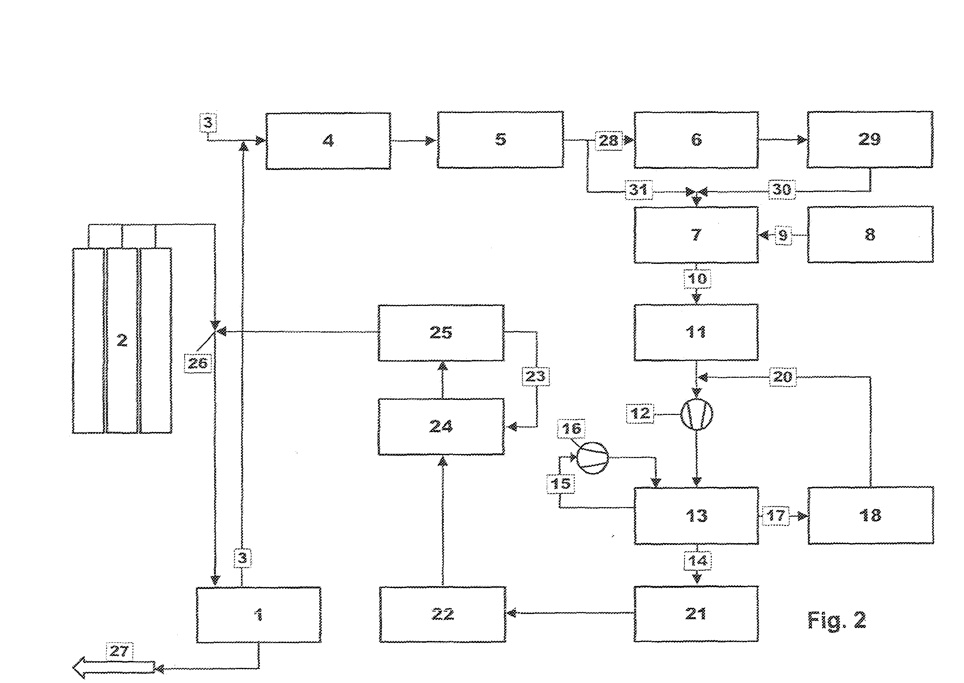

In einer zentralen Ölaufarbeitung 1 wird das aus unterschiedlichen Bohrlöchern 2 stammende Rohöl zusammengeführt, gemischt und nachfolgend einer Trenneinrichtung zugeführt, in der die wässrige Phase und gasförmige Bestandteile abgetrennt werden. Die Trenneinrichtung ist Bestandteil der zentralen Ölaufarbeitung 1. In den Figuren 1 und 2 sind symbolhaft drei Bohrlöcher 2 dargestellt.

Im Zusammenhang mit der Erdölförderung fällt Erdgas/Erdölbegleitgas mit folgender Zusammensetzung an:

| | |
|---|---|
| - Stickstoff | 1,5 % |
| - Methan | 92 % |
| - Ethan | 3,5 % |
| - Propan | 1,5 % |
| - höhere Kohlenwasserstoffe | 1 % |
| - Schwefel | 50 ppm. |

Das Erdgas/Ölbegleitgas (350.000 Nm³/h) wird in einer auf dem Gelände der Erdöllagerstätte errichteten Chemieanlage wie folgt in ein Kohlenwasserstoffgemisch umgewandelt.

### Beispiel 1

Wie in Fig. 1 gezeigt, wird Erdgas/Ölbegleitgas 3 bei einem Druck von 70 bar zunächst bei einer Temperatur von 375 °C über einem Zinkoxidbett entschwefelt (Entschwefelungseinheit 4), danach mit Prozesskondensat und Dampf gesättigt (Sättiger 5) und nach Einstellung eines Dampf-/Kohlenstoffverhältnisses von 1,0 im Prereformer 6, einem adiabatisch arbeitenden Katalysereaktor, bei 480 °C in ein Gemisch aus Methan, Kohlendioxid und Kohlenmonoxid vorgespalten.

Nach weiterer Aufheizung auf 630 bis 650 °C wird das vorgespaltene Gas einem Autothermreformer 7 zugeführt. In diesem Katalysereaktor wird durch Zusatz von auf 230 °C vorgeheiztem Sauerstoff 9, der in einer Luftzerlegungsanlage 8 gewonnen wird, bei 1030 °C ein Synthesegas 10 erzeugt, das aus Wasserstoff, Kohlenmonoxid und Kohlendioxid besteht und nur noch eine sehr kleine Menge ungespaltenes Methan enthält. Dieses Synthesegas wird in einem Abhitzesystem 11 gekühlt.

Über verschiedene Stufen, die zur Dampferzeugung bzw. Erwärmung verschiedener Gas-/Produktströme genutzt werden, wird das nunmehr bei 55 bar anliegende und gekühlte Synthesegas mit einem Kompressor 12 auf 75 bar komprimiert. Nachfolgend wird in einem dualen System, bestehend aus einem wassergekühlten und einem gasgekühlten Reaktor 13, Synthesegas katalytisch im Temperaturbereich von 220 bis 260 °C in Methanol umgewandelt und durch Kondensation ein Rohmethanol 14 mit folgender Zusammensetzung erhalten:

| | |
|---|---|
| - Methanol | 83 Gew.-% |
| - Kohlendioxid | 3,6 Gew.-% |
| - Wasser | 11,7 Gew.-% |
| - Methan | 1,5 Gew.-% |
| - höhere Kohlenwasserstoffe | 0,1 Gew.-% |
| - höhere Alkohole | 0,1 %. |

Während der Methanolsynthese wird eine Teilmenge an Synthesegas über eine Kreislaufleitung 15 im Kreislauf gefahren und dabei mittels eines weiteren Kompressors 16 auf den erforderlichen Druck gebracht. Aufgrund der im Synthesegas enthaltenen Verunreinigungen wird eine Teilmenge an Synthesegas als Puregegas 17 ausgekreist und über eine Pressure-Swing-Anlage (PSA) 18 gefahren. Dieser PSA wird auch unter hohem Druck ein Synthesegasteilstrom 19 zugeführt, der nach der Druckerhöhung mittels des Kompressors 12 abgezweigt wird. Der in der PSA 18 erzeugte Wasserstoff 20 wird auf der Saugseite des Synthesegaskompressors 12 in den Synthesegasstrom zurückgeführt.

Das nach der Methanolsynthese in mehreren Stufen kondensierte Rohmethanol 14 wird in einer nachgeschalteten Destillationsanlage 21 zunächst entgast und nachfolgend von niedrig siedenden sowie abschließend höher siedenden Produkten gereinigt. Gegenüber der klassischen dreistufigen Destillation zur Herstellung von marktfähigem Methanol wird die Destillation im Temperaturbereich von 70 bis 140 °C in nur zwei Kolonnen ausgeführt und ein Restwassergehalt im erzeugten Methanol von 4 % eingestellt. Insgesamt fallen nach der Destillation 435 t/h Rohmethanol an, die 17 t Wasser enthalten.

Das auf 4 % Wassergehalt destillierte Methanol wird nachfolgend in einem Festbettreaktor 22 (DME-Reaktor) katalytisch in ein DME (Dimethylether)/Methanol/Wasser-Gemisch umgewandelt. Das Reaktionsprodukt aus dem DME-Reaktor wird mit Recyclegas 23 zur Temperatureinstellung versetzt und nachfolgend in weiteren adiabatisch arbeitenden Reaktoren 24 im Temperaturbereich von 320 bis 420 °C in ein Kohlenwasserstoff/Wasser-Gemisch umgewandelt. Aus den eingesetzten 435 t/h Methanol entstehen dabei 191 t Kohlenwasserstoffe und 244 t Wasser. Dieses wässrige Kohlenwasserstoffgemisch wird abschließend in einer Entgasungseinheit 25 entgast und dem unaufbereiteten schweren Rohöl zugesetzt.

Gemäß diesem Beispiel werden dem unaufbereiteten schweren Rohöl (1088 t/h) kontinuierlich 435 t an wässrigem Kohlenwasserstoffgemisch mit einer Kettenlänge von vorwiegend C4 bis C12, das keine sauerstoffhaltigen Kohlenwasserstoffverbindungen enthält, pro Stunde zugemischt. Im Fließschema ist die Stelle des Zumischens mit dem Bezugszeichen 26 gekennzeichnet.

Die Zumischung des Kohlenwasserstoffgemisches erfolgt vor dem Trennprozess Rohöl/Erdölbegleitgas, der innerhalb der zentralen Ölaufbereitung 1 stattfindet.

Nachfolgend werden in der zentralen Ölaufbereitung 1 aus dem verdünnten Rohölgemisch die wässrige Phase und noch vorhandene gasförmige Bestandteile, wie Stickstoff, Kohlendioxid, Methan und Ethan, abgetrennt. Es werden 1004 t/h aufbereitetes Rohöl mit einem API 36° erhalten. Dieses kann nunmehr in herkömmlichen Transportrohrleitungen 27 mit Pumpstationen über tausende von Kilometern ohne Probleme transportiert werden. Dieses modifizierte Rohöl besitzt eine Qualität wie leichtes Rohöl.

Der Vorteil bei der weiteren Verarbeitung bzw. Raffination des leichten Rohöls zu Benzin ist, dass die zur Verbesserung der Transportfähigkeit zugesetzten speziellen Kohlenwasserstoffe absolut keinen Nachteil auf das Raffinationsverfahren haben und wirksamer Bestandteil des gewonnenen Benzins werden, wodurch sich Menge an hergestelltem Benzin um diesen Anteil erhöht.

### Beispiel 2

Die in Fig. 2 gezeigte Ausführungsvariante unterscheidet sich von der in Fig. 1 gezeigten Ausführung in folgenden Verfahrensschritten.

Nach dem Sättiger 5 wird über eine erste Leitung 28 ein erster Teilstrom (Mengenanteil etwa 40 %) des mit Wasser gesättigten, entschwefelten Prozessgases mit Dampf gemischt und mit einer Temperatur von etwa 480 °C dem Prereformer 6 zugeführt.

In diesem wird das Prozessgas in ein Gemisch aus Methan, Kohlendioxid, Wasserstoff und Kohlenmonoxid vorgespalten. Nach weiterer Aufheizung auf 520 °C gelangt das vorgespaltene Prozessgas in einen Steamreformer 29, einem außenbeheizten Röhrenreaktor mit Nickel-Katalysator, und wird in diesem in ein erstes Synthesegas 30, ein Gemisch aus Wasserstoff, CO und CO₂, umgewandelt.

Dieses erste Synthesegas 30 wird zu dem in der anderen, zweiten Leitung 31 geführten Teilstrom (Menge ca. 60 %) des mit Wasser gesättigten, entschwefelten Prozessgases, das nach dem Sättiger 5 anfällt, zurückgeführt, mit diesem vermischt und mit einer Mischtemperatur von 670 °C dem Autothermreformer 7 zugeführt.

Die Aufteilung des Prozessgases in zwei Teilströme kann entweder vor, nach dem Sättiger 7, oder nach dem Prereformer 6 vorgenommen werden.

Im Autothermreformer 7, einem adiabatisch arbeitenden Katalysereaktor, wird das Mischgas durch Zusatz von auf 240 °C erwärmten Sauerstoff 9, der in einer Luftzerlegungsanlage 8 gewonnen wird, bei 980 °C vollständig in ein zweites Synthesegas 10' umgewandelt, das nur noch eine sehr kleine Menge ungespaltenes Methan enthält. Dieses Synthesegas wird in dem nachfolgenden Abhitzesystem 11 gekühlt.

Das mit einem Druck von 32 bar anliegende Synthesegas wird nachfolgend in analoger Weise, wie in Beispiel 1 angegeben, weiterbehandelt, um ein wasserhaltiges Kohlenwasserstoffgemisch mit einer Kettenlänge von vorwiegend C4 bis C12 herzustellen, mit dem einzigen Unterschied, dass nach dem Kompressor 12 kein Synthesegasteilstrom 19 abgezweigt und der Pressure-Swing-Anlage (PSA) 18 zugeführt wird.

Mit dieser Verfahrensvariante ist es möglich, im Vergleich zu der Verfahrensweise gemäß Beispiel 1, den Gasverbrauch für die Herstellung des wasserhaltigen Kohlenwasserstoffgemisches um ca. 10 % zu reduzieren.

## Patentansprüche

1. Verfahren zur Verbesserung der Transportfähigkeit von schwerem Rohöl, wobei dem schweren Rohöl ein viskositätserniedrigendes Mittel zugesetzt wird, **dadurch gekennzeichnet, dass** das Mittel ein wasserhaltiges Kohlenwasserstoffgemisch mit einer Kettenlänge von vorwiegend C4 bis C12 ist, das keine sauerstoffhaltigen Kohlenwasserstoffverbindungen enthält, und im Bereich einer Erdöllagerstätte aus anfallendem Erdgas und/oder Erdölbegleitgas mit folgenden Verfahrensschritten hergestellt wird:
a) Umwandlung des Erdgases und/oder Erdölbegleitgases in ein Methanol/Wasser-Gemisch,
b) destillative Aufarbeitung des Methanol-Wasser-Gemisches zu einem Destillat mit einem hohen Wasser- und Alkoholgehalt von über 90 %,
c) katalytische Umwandlung des Destillates in ein Dimethylether/Methanol/Wasser-Gemisch,
d) Umwandlung des Dimethylether/Methanol/Wasser-Gemisches durch Dehydratation in das wasserhaltige Kohlenwasserstoffgemisch mit einer Kettenlänge C4 bis C12;
und das gemäß den Verfahrensschritten a) bis d) erhaltene Kohlenwasserstoffgemisch entweder unbehandelt oder nach Entgasung und/oder Entwässerung dem schweren Rohöl zugesetzt wird, wodurch aus dem schweren Rohöl ein in seiner Qualität leichtes Rohöl erhalten wird, das über Leitungen zu einer Raffinerie transportiert wird und während der nachfolgenden Raffination des leichten Rohöls zu herkömmlichem Benzin die produzierte Menge an Benzin um die im wasserhaltigen Kohlenwasserstoffgemisch enthaltenen Kohlenwasserstoffe erhöht wird.

2. Verfahren nach Anspruch 1, **dadurch gekennzeichnet, dass** die Umwandlung des Erd- und/oder Erdölbegleitgases in ein Methanol-Wasser-Gemisch mittels folgender Verfahrensschritte vorgenommen wird:
- Entschwefelung;
- Sättigung mit Prozesskondensat und Dampf;
- Vorspaltung in ein Gasgemisch aus Methan, Kohlendioxid und Kohlenmonoxid;
- anschließend wird das vorgespaltetene Gasgemisch unter erhöhter Temperatur und bei einem Druck von mindestens 50 bar in einem Autothermreaktor katalytisch in Synthesegas unter Zusatz von vorgeheiztem Sauerstoff umgewandelt, das abgekühlt und mittels eines Kompressors komprimiert wird, und
- nachfolgend aus diesem durch katalytische Umwandlung im Rahmen einer zweistufigen Methanol-Synthese in einem wassergekühlten und in einem gasgekühlten Reaktor Methanol gewonnen und durch nachfolgende mehrstufige Kondensation Rohmethanol (Methanol-Wasser-Gemisch) erhalten wird.

3. Verfahren nach Anspruch 1, **dadurch gekennzeichnet, dass** die Umwandlung des Erd- und/oder Erdölbegleitgases in ein Methanol-Wasser-Gemisch mittels folgender Verfahrensschritte vorgenommen wird:
- Entschwefelung;
- Sättigung mit Prozesskondensat und Dampf;
- nachfolgende Auskreisung eines Teilstromes an mit Wasser gesättigtem Prozessgas, das in ein Gasgemisch aus Methan, Wasserstoff, Kohlendioxid und Kohlenmonoxid vorgespalten wird;
- dieses Gasgemisch wird in einem Steamreformer in ein erstes Synthesegas, ein Gemisch aus Wasserstoff, Kohlendioxid und Kohlenmonoxid, umgewandelt, das wieder in den mit Wasser gesättigten Prozessgasstrom zurückgeführt und mit diesem vermischt wird;
- anschließend wird der Prozessgasstrom unter erhöhter Temperatur und bei einem Druck von mindestens 50 bar in einem Autothermreaktor unter Zusatz von vorgeheiztem Sauerstoff katalytisch in ein zweites Synthesegas umgewandelt, das abgekühlt und mittels eines Kompressors komprimiert wird, und
- nachfolgend aus diesem durch katalytische Umwandlung im Rahmen einer zweistufigen Methanol-Synthese in einem wassergekühlten und in einem gasgekühlten Reaktor Methanol gewonnen und durch nachfolgende mehrstufige Kondensation Rohmethanol (Methanol-Wasser-Gemisch) erhalten wird.

4. Verfahren nach Anspruch 3, **dadurch gekennzeichnet, dass** das wassergesättigte Prozessgas nach dem Prereformer in zwei Teilströme aufgeteilt wird, wobei der eine Teilstrom zum Steamreformer und der andere Teilstrom zum Autothermreformer gefahren wird.

5. Verfahren nach einem der Ansprüche 1 bis 4, **dadurch gekennzeichnet, dass** das erhaltene Methanol-Wasser-Gemisch (Rohmethanol) einer zweitstufigen Destillation unterzogen wird, wobei in der ersten Stufe niedrig siedende und in der zweiten Stufe höher siedende Verbindungen abgetrennt werden und ein Destillat mit einem hohen Wasser- und Alkoholgehalt entsteht, das nachfolgend in einem Festbettreaktor katalytisch in ein Dimethylether/Methanol/Wasser-Gemisch umgewandelt wird, das anschließend in weiteren adiabatisch arbeitenden Reaktoren im Temperaturbereich von 300 bis 450 °C in das wasserhaltige Kohlenwasserstoffgemisch als Endprodukt umgewandelt wird.

6. Verfahren nach einem der Ansprüche 1 bis 5, **dadurch gekennzeichnet, dass** als Zwischenprodukt ein Methanol mit einem Restwassergehalt von mindestens 4 % und einem Alkoholgehalt von 0,1 % gebildet wird.

7. Verfahren nach einem der Ansprüche 1 bis 6, **dadurch gekennzeichnet, dass** das im Festbettreaktor anfallende Dimethylether/Methanol/Wasser-Gemisch mit Recyclegas zur Temperatureinstellung versetzt wird.

8. Verfahren nach einem der Ansprüche 1 bis 7, **dadurch gekennzeichnet, dass** eine erste Teilmenge an Synthesegas ausgekreist, im Kreislauf gefahren und dabei auf den erforderlichen Betriebsdruck komprimiert wird.

9. Verfahren nach einem der Ansprüche 1 bis 8, **dadurch gekennzeichnet, dass** eine zweite Teilmenge an Synthesegas ausgekreist, in einer Pressure-Swing-Anlage Wasserstoff abgetrennt wird, der auf der Saugseite des Kompressors in den Synthesegasstrom eingeleitet wird.

10. Verfahren nach einem der Ansprüche 1 bis 9, **dadurch gekennzeichnet, dass** das gebildete Kohlenwasserstoffgemisch unmittelbar, im Bereich der Erdöllagerstätte, entweder dem bereits geförderten und/oder dem noch unterirdisch gelagerten schweren Rohöl über das Bohrloch zugeführt wird.

11. Verfahren nach einem der Ansprüche 1 bis 10, **dadurch gekennzeichnet, dass** das gebildete Kohlenwasserstoffgemisch über ein Spülrohr in das Bohrloch eingebracht wird.

12. Verfahren nach einem der Ansprüche 1 bis 11, **dadurch gekennzeichnet, dass** das gebildete Kohlenwasserstoffgemisch vor dem Inkontaktbringen mit dem schweren Rohöl entwässert und entgast wird.

13. Verfahren nach einem der Ansprüche 1 bis 12, **dadurch gekennzeichnet, dass** aufbereitetes Kohlenwasserstoffgemisch, das entwässert und entgast ist, vor oder nach der zentralen Ölaufarbeitung dem schweren Rohöl zugesetzt wird.

14. Verfahren nach einem der Ansprüche 1 bis 12, **dadurch gekennzeichnet, dass** nicht aufbereitetes Kohlenwasserstoffgemisch vor der zentralen Ölaufarbeitung dem schweren Rohöl zugesetzt wird.

15. Verfahren nach einem der Ansprüche 1 bis 14, **dadurch gekennzeichnet, dass** nach der Abtrennung von Wasser und Ölbegleitgas aus dem Rohöl diesem eine weitere Menge an aufbereitetem Kohlenwasserstoffgemisch zugesetzt wird, die in Abhängigkeit von der Viskosität des Schweröls so dosiert wird, dass ein leichtes Rohöl gebildet wird.

16. Verfahren nach einem der Ansprüche 1 bis 15, **dadurch gekennzeichnet, dass** aufbereitetes Kohlenwasserstoffgemisch auf der Saugseite der zum Transport des Rohöls eingesetzten Pumpe zugeführt wird.

17. Verfahren nach einem der Ansprüche 1 bis 16, **dadurch gekennzeichnet, dass** die Viskosität des geförderten schweren Rohöls gemessen und in Abhängigkeit vom aktuellen Messergebnis die Menge an Kohlenwasserstoffgemisch dosiert zugesetzt wird, um leichtes Rohöl zu erhalten.

18. Verfahren nach einem der Ansprüche 1 bis 17, **dadurch gekennzeichnet, dass** aufbereitetes Kohlenwasserstoffgemisch und schweres Rohöl in einer separaten Mischeinrichtung vermischt werden.

## Claims

1. A method for improving the transportability of heavy crude oil, wherein a viscosity-reducing agent is added to the heavy crude oil, **characterized in that** the agent is a water-containing hydrocarbon mixture with a chain length of predominantly C4 to C12, which does not contain any oxygen-containing hydrocarbon compounds, and is produced in the area of an oil deposit from arising natural gas and/or crude oil-associated gas with the following method steps:
a) conversion of the natural gas and/or crude oil-associated gas into a methanol/water mixture,
b) processing by distillation of the methanol/water mixture to form a distillate having a high water and alcohol content of above 90%,
c) catalytic conversion of the distillate into a dimethyl ether/methanol/water mixture,
d) conversion of the dimethyl ether/methanol/water mixture by dehydration into the water-containing hydrocarbon mixture with a chain length C4 to C12;
and the hydrocarbon mixture obtained according to the method steps a) to d) is added either untreated or after degassing and/or dewatering to the heavy crude oil, as a result of which, from the heavy crude oil, a crude oil light in quality is obtained which is transported via lines to a refinery and, during the subsequent refining of the light crude oil into conventional gasoline, the amount of gasoline produced is increased by the hydrocarbons contained in the water-containing hydrocarbon mixture.

2. The method according to claim 1, **characterized in that** the conversion of the natural gas and/or crude oil-associated gas into a methanol/water mixture is carried out by means of the following method steps:
- desulfurization;
- saturation with process condensate and steam;
- pre-cracking into a gas mixture of methane, carbon dioxide and carbon monoxide;
- then, the pre-cracked gas mixture is catalytically converted into synthesis gas at an elevated temperature and a pressure of at least 50 bar in an autothermal reactor while adding preheated oxygen, which synthesis gas is cooled and compressed by means of a compressor, and
- subsequently, by catalytic conversion in the context of a two-stage methanol synthesis in a water-cooled and in a gas-cooled reactor, methanol is obtained therefrom, and crude methanol (methanol/water mixture) is obtained by subsequent multi-stage condensation.

3. The method according to claim 1, **characterized in that** the conversion of the natural gas and/or crude oil-associated gas into a methanol/water mixture is carried out by means of the following method steps:
- desulfurization;
- saturation with process condensate and steam;
- subsequent removal from the circuit of a sub-stream of water-saturated process gas which is pre-cracked into a gas mixture of methane, hydrogen, carbon dioxide and carbon monoxide;
- this gas mixture is converted in a steam reformer into a first synthesis gas, a mixture of hydrogen, carbon dioxide and carbon monoxide, which is reintroduced to the water-saturated process gas stream and mixed therewith;
- then, the process gas stream is catalytically converted, at an elevated temperature and a pressure of at least 50 bar in an autothermal reactor while adding preheated oxygen, into a second synthesis gas which is cooled and compressed by means of a compressor, and
- subsequently, by catalytic conversion in the context of a two-stage methanol synthesis in a water-cooled and in a gas-cooled reactor, methanol is obtained therefrom, and crude methanol (methanol/water mixture) is obtained by subsequent multi-stage condensation.

4. The method according to claim 3, **characterized in that** the water-saturated process gas, after the pre-reformer, is divided into two sub-streams, wherein the one sub-stream is run to the steam reformer and the other sub-stream is run to the autothermal reformer.

5. The method according to any one of the claims 1 to 4, **characterized in that** the methanol/water mixture (crude methanol) obtained is subjected to a two-stage distillation, wherein, in the first stage, low-boiling compounds are separated off, and in the second stage higher-boiling compounds are separated off, and a distillate having a high water and alcohol content is formed, which is then catalytically converted in a fixed-bed reactor into a dimethyl ether/methanol/water mixture which is then converted in further adiabatically operating reactors in the temperature range from 300 to 450°C into the water-containing hydrocarbon mixture as an end product.

6. The method according to any one of the claims 1 to 5, **characterized in that** a methanol having a residual water content of at least 4%, and an alcohol content of 0.1 %, is formed as an intermediate product.

7. The method according to any one of the claims 1 to 6, **characterized in that** the dimethyl ether/methanol/water mixture arising in the fixed-bed reactor is admixed with recycled gas for temperature adjustment.

8. The method according to any one of the claims 1 to 7, **characterized in that** a first sub-quantity of synthesis gas is removed from the circuit, circulated, and in the process compressed to the required operating pressure.

9. The method according to any one of the claims 1 to 8, **characterized in that** a second sub-quantity of synthesis gas is removed from the circuit, hydrogen is separated off in a pressure-swing facility, which hydrogen is introduced into the synthesis gas stream on the suction side of the compressor.

10. The method according to any one of the claims 1 to 9, **characterized in that** the hydrocarbon mixture formed is fed directly in the area of the oil deposit either to the heavy crude oil already produced and/or via the borehole to the heavy crude oil still stored underground.

11. The method according to any one of the claims 1 to 10, **characterized in that** the hydrocarbon mixture formed is introduced into the borehole via a purge tube.

12. The method according to any one of the claims 1 to 11, **characterized in that** the hydrocarbon mixture formed is dewatered and degassed before it is brought into contact with the heavy crude oil.

13. The method according to any one of the claims 1 to 12, **characterized in that** treated hydrocarbon mixture that is dewatered and degassed is added to the heavy crude oil before or after the central oil processing.

14. The method according to any one of the claims 1 to 12, **characterized in that** non-treated hydrocarbon mixture is added to the heavy crude oil before the central oil processing.

15. The method according to any one of the claims 1 to 14, **characterized in that**, after separating off water and oil-associated gas from the crude oil, a further amount of treated hydrocarbon mixture is added thereto, which, depending on the viscosity of the heavy oil, is metered in such a manner that a light crude oil is formed.

16. The method according to any one of the claims 1 to 15, **characterized in that** treated hydrocarbon mixture is supplied on the suction side of the pump used for the transport of the crude oil.

17. The method according to any one of the claims 1 to 16, **characterized in that** the viscosity of the extracted heavy crude oil is measured, and, depending on the current measurement result, the amount of hydrocarbon mixture is added in a metered manner in order to obtain light crude oil.

18. The method according to any one of the claims 1 to 17, **characterized in that** treated hydrocarbon mixture and heavy crude oil are mixed in a separate mixing device.

## Revendications

1. Procédé destiné à améliorer la transportabilité de pétrole brut lourd, dans lequel un agent réducteur de viscosité est ajouté au pétrole brut lourd, **caractérisé par le fait que** ledit agent est un mélange d'hydrocarbures contenant de l'eau et ayant une longueur de chaîne principalement en C4 à C12 qui ne contient pas de composés d'hydrocarbures contenant de l'oxygène, et est préparé, au niveau d'un gisement de pétrole, à partir de gaz naturel produit et/ou de gaz associé au pétrole, par les étapes de procédé suivantes:
a) transformation du gaz naturel et/ou du gaz associé au pétrole en un mélange méthanol-eau,
b) traitement distillatoire du mélange méthanol-eau pour obtenir un distillat ayant une forte teneur en eau et en alcool supérieure à 90 %,
c) transformation catalytique du distillat en un mélange de diméthyléther/méthanol/eau,
d) transformation, par déshydratation, du mélange de diméthyléther/méthanol/eau pour obtenir ledit mélange d'hydrocarbures contenant de l'eau et ayant une longueur de chaîne de C4 à C12 ;
et que le mélange d'hydrocarbures obtenu selon les étapes de procédé a) à d) est ajouté au pétrole brut lourd soit sans être traité soit après le dégazage et/ou la déshydratation, ce par quoi on obtient à partir du pétrole brut lourd un pétrole brut léger en qualité qui est transporté par des conduites à une raffinerie, et, pendant l'affinage subséquent du pétrole brut léger destiné à obtenir de l'essence conventionnelle, la quantité produite d'essence est augmentée des hydrocarbures contenus dans le mélange d'hydrocarbures contenant de l'eau.

2. Procédé selon la revendication 1, **caractérisé par le fait que** la transformation du gaz naturel et/ou du gaz associé au pétrole en un mélange méthanol-eau est mise en oeuvre au moyen des étapes de procédé suivantes :
- désulfuration;
- saturation avec du condensat de processus et de la vapeur;
- pré-séparation en un mélange gazeux de méthane, de dioxyde de carbone et de monoxyde de carbone;
- ensuite, le mélange gazeux pré-séparé est transformé façon catalytique, à température élevée et à une pression de 50 bars au moins, dans un réacteur autothermique, en ajoutant de l'oxygène préchauffé, en gaz de synthèse qui est refroidi et comprimé en utilisant un compresseur, et,
- ensuite, on obtient à partir de celui-ci du méthanol par transformation catalytique, dans le cadre d'une synthèse de méthanol à deux étapes, dans un réacteur refroidi à l'eau et un réacteur refroidi au gaz, et on obtient du méthanol brut (mélange méthanol-eau) par une condensation subséquente à plusieurs étapes.

3. Procédé selon la revendication 1, **caractérisé par le fait que** la transformation du gaz naturel et/ou du gaz associé au pétrole en un mélange méthanol-eau est mise en oeuvre au moyen des étapes de procédé suivantes consistant à:
- désulfurer;
- saturer avec du condensat de processus et de la vapeur;
- ensuite, à retirer du circuit un courant partiel de gaz de processus saturé avec de l'eau qui est pré-séparé en un mélange gazeux de méthane, d'hydrogène, de dioxyde de carbone et de monoxyde de carbone;
- ce mélange gazeux est transformé dans un reformeur à la vapeur en un premier gaz de synthèse, un mélange d'hydrogène, de dioxyde de carbone et de monoxyde de carbone, qui est ramené au courant de gaz de processus saturé avec de l'eau et est mélangé à celui-ci;
- puis, le courant de gaz de processus est transformé de façon catalytique, à température élevée et à une pression de 50 bars au moins, dans un réacteur autothermique, en ajoutant de l'oxygène préchauffé, en un deuxième gaz de synthèse qui est refroidi et comprimé en utilisant un compresseur, et,
- ensuite, on obtient à partir de celui-ci du méthanol par transformation catalytique, dans le cadre d'une synthèse de méthanol à deux étapes, dans un réacteur refroidi à l'eau et un réacteur refroidi au gaz, et on obtient du méthanol brut (mélange méthanol-eau) par une condensation subséquente à plusieurs étapes.

4. Procédé selon la revendication 3, **caractérisé par le fait que** le gaz de processus saturé avec de l'eau est divisé, en aval du pré-reformeur, en deux courants partiels, l'un des courants partiels étant amené au reformeur à la vapeur et l'autre courant partiel étant amené au reformeur autothermique.

5. Procédé selon l'une quelconque des revendications 1 à 4, **caractérisé par le fait que** le mélange méthanol-eau obtenu (méthanol brut) est soumis à une distillation à deux étapes, dans lequel, dans la première étape, on sépare des composés à bas point d'ébullition et, dans la deuxième étape, des composés à point d'ébullition plus élevé, et on obtient un distillat à forte teneur en eau et en alcool qui, ensuite, est transformé façon catalytique, dans un réacteur à lit fixe, en un mélange de diméthyléther/méthanol/eau qui est transformé ensuite, dans d'autres réacteurs fonctionnant de façon adiabatique, dans la plage de températures allant de 300 à 450 °C, pour obtenir le mélange d'hydrocarbures contenant de l'eau, en tant que produit final.

6. Procédé selon l'une quelconque des revendications 1 à 5, **caractérisé par le fait qu'**un méthanol ayant une teneur en eau résiduelle de 4 % au moins et une teneur en alcool de 0,1 % est formé en tant qu'intermédiaire.

7. Procédé selon l'une quelconque des revendications 1 à 6, **caractérisé par le fait que** le mélange de diméthyléther/méthanol/eau produit dans le réacteur à lit fixe est mélangé avec du gaz de recyclage pour le réglage de la température.

8. Procédé selon l'une quelconque des revendications 1 à 7, **caractérisé par le fait qu'**une première partie de gaz de synthèse est retirée du circuit, est mise en circulation et est ainsi comprimée à la pression de fonctionnement requise.

9. Procédé selon l'une quelconque des revendications 1 à 8, **caractérisé par le fait qu'**une deuxième partie de gaz de synthèse est retirée du circuit, que de l'hydrogène est séparé dans une installation à inversion de pression (pressure swing) qui est introduit sur le côté d'aspiration du compresseur dans le courant de gaz de synthèse.

10. Procédé selon l'une quelconque des revendications 1 à 9, **caractérisé par le fait que** le mélange d'hydrocarbures formé est amené directement, au niveau du gisement de pétrole, par le trou de sondage, soit au pétrole brut lourd déjà produit et/ou au pétrole brut lourd stocké encore sous terre.

11. Procédé selon l'une quelconque des revendications 1 à 10, **caractérisé par le fait que** le mélange d'hydrocarbures formé est introduit par l'intermédiaire d'un tuyau de rinçage dans le trou de sondage.

12. Procédé selon l'une quelconque des revendications 1 à 11, **caractérisé par le fait que** le mélange d'hydrocarbures formé est déshydraté et dégazé avant la mise en contact avec le pétrole brut lourd.

13. Procédé selon l'une quelconque des revendications 1 à 12, **caractérisé par le fait que** du mélange d'hydrocarbures traité qui est déshydraté et dégazé est ajouté au pétrole brut lourd avant ou après le traitement central d'huile.

14. Procédé selon l'une quelconque des revendications 1 à 12, **caractérisé par le fait que** du mélange d'hydrocarbures non traité est ajouté au pétrole brut lourd avant le traitement central d'huile.

15. Procédé selon l'une quelconque des revendications 1 à 14, **caractérisé par le fait que**, après avoir séparé l'eau et le gaz associé à l'huile du pétrole brut, on ajoute à ce dernier une autre quantité de mélange d'hydrocarbures traité qui est dosée en fonction de la viscosité de l'huile lourde de telle manière qu'un pétrole brut léger est formé.

16. Procédé selon l'une quelconque des revendications 1 à 15, **caractérisé par le fait que** du mélange d'hydrocarbures traité est amené sur le côté d'aspiration à la pompe utilisée pour le transport du pétrole brut.

17. Procédé selon l'une quelconque des revendications 1 à 16, **caractérisé par le fait que** la viscosité du pétrole brut lourd produit est mesurée et que la quantité de mélange d'hydrocarbures est ajoutée de manière dosée en fonction du résultat actuel de mesure afin d'obtenir du pétrole brut léger.

18. Procédé selon l'une quelconque des revendications 1 à 17, **caractérisé par le fait que** du mélange d'hydrocarbures traité et du pétrole brut lourd sont mélangés dans un dispositif mélangeur séparé.
